# DEMANDE DE BREVET EUROPEEN

(11) **EP 2 567 673 A1**
(43) Date de publication de la demande: **13.03.2013**
(21) Numéro de dépôt: 12183822.1
(22) Date de dépôt: 11.09.2012
(51) Int. Cl.: A61F 2/08, A61F 2/00

(54) **Bande de renfort pour la restauration d'un tissu mou**

(30) Priorité: 20.09.2011 FR 1158359; 12.09.2011 US 201113230623
(71) Demandeur: Tornier, Inc., Bloomington, MN 55437 (US)
(72) Inventeur: Peterson, Dale, LA JOLLA (CA), CA California 92037 (US); Anderson, Justin, MINNETONKA - USA, CA California 55345 (US); Ohashi, Kevin, Jamaican PLain, CA California 02130 (US); Mattern, Ralph, SAN DIEGO - (CA), CA California 92129 (US); Rushdy, Jamal, EDEN PRAIRIE - (MN), CA California 55347 (US)
(74) Mandataire: Grand, Guillaume

(57) **Abrégé**

Cette bande de renfort (1) comprend un élément textile allongé (10) qui, suivant sa direction longitudinale, présente au moins un bord libre (12, 13) qui est replié dans un volume intérieur de l'élément textile de manière que l'élément textile présente une partie longitudinale pliée sur elle-même (15, 16), laquelle partie pliée sur elle-même forme, en service, une extrémité de la bande de renfort et est attachée à un fil de suture rapporté (20, 30) qui est adapté pour être fixé à un os. Selon l'invention, l'élément textile comprend, voire consiste en, des monofilaments et des fils multifilaments, qui sont entrelacés les uns avec les autres, en particulier en définissant une porosité de colonisation cellulaire, et qui, au niveau du ou de chaque bord libre (12, 13) de l'élément textile (10), sont fusionnés. La résistance mécanique de la bande et sa capacité de fixation, tant primaire que secondaire, s'en trouvent renforcées.

## Description

La présente invention concerne une bande de renfort pour la restauration d'un tissu mou, tel qu'un tendon ou un ligament.

Quand un tissu mou d'un être humain, tel qu'un tendon ou un ligament, est endommagé dans sa région d'attache à un os, la liaison entre ce tissu mou et l'os peut être rétablie de différentes manières, par exemple avec des éléments de suture et des vis. Les techniques chirurgicales correspondantes sont souvent longues et délicates à mettre en oeuvre. De plus, malgré le soin apporté par le chirurgien au cours de l'intervention, les risques de séparation de la suture, en particulier au niveau du tissu mou, sont souvent élevés car, généralement, les moyens de suture utilisés pour réattacher le tissu mou avec l'os affaiblissent le tissu mou et/ou l'os. Ceci est particulièrement critique dans le contexte du rétablissement de la liaison à un os des tendons et des ligaments appartenant à la coiffe des rotateurs de l'épaule, en raison des fortes contraintes mécaniques auxquelles sont soumis ces ligaments et ces tendons lors des mouvements de l'épaule.

Par ailleurs, mettre en oeuvre les techniques précitées par arthroscopie est particulièrement difficile si bien que de nombreuses restaurations échouent lorsque le chirurgien cherche à faire passer les éléments de suture à travers les tissus mous. Un patch de renfort est donc souvent utilisé, ce qui augmente généralement la durée de l'intervention, en demandant un effort opératoire significatif pour le chirurgien. Ainsi, il n'est pas rare que des interventions de ce type demandent 40 à 90 minutes supplémentaires au chirurgien pour mettre en place un tel patch de renfort.

Ceci étant rappelé, l'invention s'intéresse plus particulièrement à la restauration de tissus mous, en utilisant une bande de renfort textile. Généralement, pour présenter une résistance à l'arrachement suffisante, ces bandes textiles comprennent des anneaux ou des rondelles permettant de fixer la bande au tissu mou et/ou à l'os à l'aide de vis ou d'éléments d'ancrage similaires, introduits dans les anneaux ou rondelles précités. Il en résulte que la géométrie de ces bandes de renfort est prédéfinie, sans permettre d'adapter les dimensions de la bande de renfort, notamment aux spécificités du site d'implantation de cette bande.

Pour répondre à cette problématique, WO-A-2010/105171, sur lequel est basé le préambule de la revendication 1, propose de réaliser une bande de renfort sous forme d'un élément textile allongé dont les bords libres opposés sont repliés dans un volume intérieur de l'élément textile. L'élément textile présente alors, entre sa partie longitudinale courante et chacun des bords libres ainsi repliés, une partie longitudinale pliée sur elle-même, qui est prévue pour être reliée au tissu mou par un fil de suture. Grâce à cette bande de renfort, le chirurgien dispose d'une solution résistante et intègre, dont il peut aisément adapter la dimension longitudinale, et ce avant ou pendant l'intervention.

Le but de la présente invention est d'améliorer la bande de renfort proposée dans WO-A-2010/105171, notamment pour renforcer sa résistance mécanique et sa capacité de fixation tant primaire que secondaire.

A cet effet, l'invention a pour objet une bande de renfort pour la restauration d'un tissu mou, telle que définie à la revendication 1.

Une des idées à la base de l'invention a été de mettre au point, pour une bande de restauration du type de celle proposée dans WO-A-2010/105171, une structure textile qui concilie une haute résistance à l'arrachement, notamment au niveau de sa liaison entre l'élément textile et le fil de suture, et une capacité de colonisation cellulaire sur le long terme, même lorsque la bande de renfort est mise sous tension au cours ou après son implantation. Selon l'invention, on prévoit que l'élément textile de la bande de renfort comporte, voire consiste en un entrelacement de monofilaments et de fils multifilaments. De cette façon, la structure de cet élément textile présente une bonne résistance mécanique et peut être passée à travers et/ou pressée contre un tissu mou sans craindre son endommagement, avantageusement tout en maintenant une porosité suffisante, c'est-à-dire la présence d'espaces libres entre les monofilaments et les fils muiltifilaments, pour que l'élément textile soit progressivement colonisé par croissance du tissu mou environnant, même lorsque la bande de renfort est sous tension. En plus de ces avantages, qui concernent plus spécifiquement la partie courante principale de la bande, la partie pliée sur elle-même de l'élément textile, prévue à au moins l'une ou aux deux extrémités de la bande de renfort, présente l'avantage de pouvoir être attachée de manière particulièrement résistante à un fil de suture à fixer à un os. En effet, au niveau de la ou chaque partie pliée sur elle-même de l'élément textile, quatre « épaisseurs » de monofilaments et de fils multifilaments se superposent, étant remarqué que, dans les deux épaisseurs agencées à l'intérieur du volume intérieur de l'élément textile, les monofilaments et les fils multifilaments sont, au niveau du bord libre de l'élément textile, fusionnés pour stabiliser leur structure et, par là, pérenniser leur comportement mécanique de coopération avec les deux épaisseurs extérieures, sous l'action de leur attachement relatif par le fil de suture. Par la suite, divers modes de réalisation sont présentés en ce qui concerne le mode d'attachement de ce fil de suture à la ou chaque partie pliée sur elle-même de l'élément textile. Dans tous les cas, grâce à la haute résistance mécanique de cet attachement entre le fil de suture et l'extrémité correspondante de la bande de renfort, ce fil de suture peut avantageusement être utilisé pour manoeuvrer, tracter et/ou ancrer la bande, notamment en coopérant avec des matériels ad hoc dont les performance de fixation sont meilleures avec un tel fil de suture, qu'avec des portions de bande textile larges.

Des caractéristiques additionnelles avantages de la bande de renfort conforme à l'invention, prises isolément ou selon toutes les combinaisons techniquement possibles, sont spécifiées aux revendications dépendantes 2 et suivantes.

L'invention sera mieux comprise à la lecture de la description qui va suivre, donnée uniquement à titre d'exemple et faite en se référant aux dessins sur lesquels :
- la figure 1 est une vue schématique en élévation d'une bande de renfort selon un premier mode de réalisation conforme à l'invention ;
- la figure 2 est une vue schématique en perspective montrant, à grande échelle, la structure textile d'une zone cerclée II sur la figure 1 ;
- la figure 3 est une coupe longitudinale schématique d'une partie de la bande de la figure 1 ; et
- la figure 4 est une vue similaire à la figure 3, illustrant un deuxième mode de réalisation conforme à l'invention.

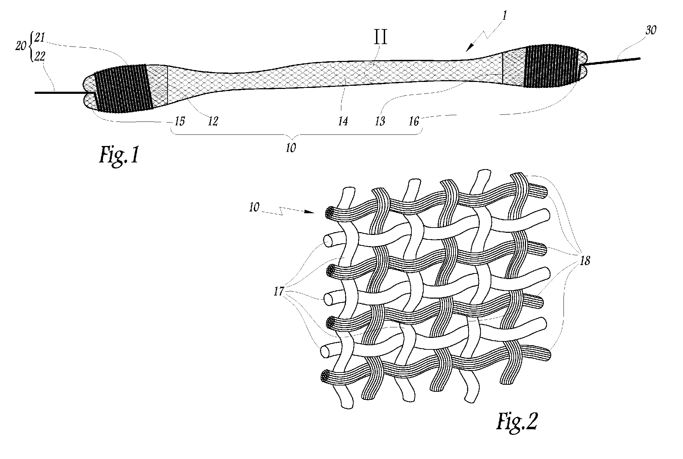

Sur les figures 1 à 3 est représentée une bande de renfort 1 pour la restauration d'un tissu mou, non représenté, tel qu'un tendon ou un ligament. A titre d'exemple du contexte d'utilisation potentielle de cette bande de renfort 1, le lecteur pourra se reporter au document précité WO-A-2010/105171.

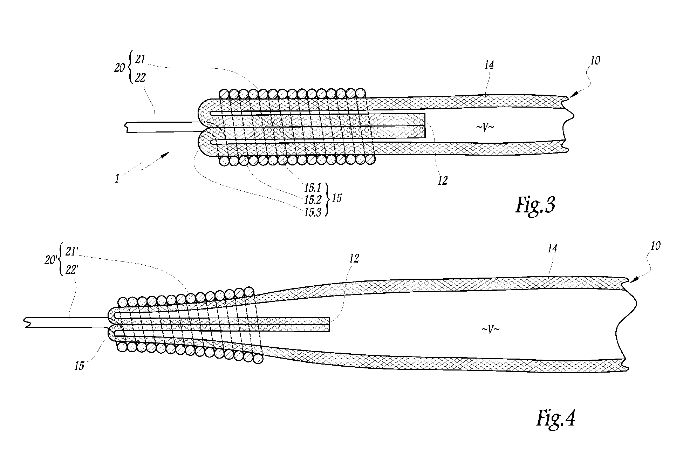

Comme bien visible sur les figures 1 et 3, la bande 1 comprend un élément textile 10, qui présente une forme allongée suivant la dimension principale de la bande 1. Dans l'exemple de réalisation considéré ici, l'élément textile 10 présente ainsi une forme globalement tubulaire, délimitant intérieurement un volume V globalement cylindrique, étant remarqué que, eu égard à la souplesse de la structure textile de l'élément 10, les formes géométriques précitées ne sont pas définies de manière rigoureusement figées, mais sont sujettes à des variations, le cas échéant significatives, liées aux déformations appliquées en service à l'élément textile 10.

Lorsqu'on parcourt l'élément textile 10 suivant sa direction longitudinale, cet élément s'étend entre deux bords libres opposés, respectivement référencés 12 et 13 sur la figure 1, seul le bord libre 12 étant visible à la figure 3. Chacun de ces bords libres 12 et 13 est replié dans le volume intérieur V, comme bien visible sur la figure 3. De cette façon, suivant la direction longitudinale de l'élément textile 10, chacun des bords libres 12 et 13 est relié à la partie longitudinale courante 14 de cet élément 10, à l'intérieur de laquelle la partie correspondante du volume V est libre, c'est-à-dire non occupée par les bords libres 12 et 13, par une partie longitudinale 15, 16 de l'élément textile, qui est pliée sur elle-même, seule la partie pliée sur elle-même 15 étant visible à la figure 3. En coupe longitudinale comme sur la figure 3, chacune de ces parties pliées sur elles-mêmes 15 et 16 présente un profil globalement en M : ainsi, comme montré pour la partie pliée sur elle-même 15 sur la figure 3, cette partie 15 inclut, d'une part, une sous-partie 15.1, qu'on peut qualifier de sous-partie intérieure et qui forme, vis-à-vis du reste de l'élément textile 10, un ourlet agencé à l'intérieur du volume V, plus précisément à l'intérieur de l'extrémité, suivant la dimension principale de la bande 1, de ce volume V, et, d'autre part, une sous-partie 15.2, qu'on peut qualifier de sous-partie extérieure et qui s'étend globalement dans le prolongement de la partie courante 14, les sous-parties 15.1 et 15.2 étant liées l'une à l'autre par une sous-partie 15.3 de la partie 15, coudée à 180° environ. Ainsi, suivant la dimension principale de la bande de renfort 1, les parties pliées sur elles-mêmes 15 et 16 de l'élément textile 10 forment les extrémités opposées de la bande 1, en fermant le volume intérieur V.

Comme expliqué en détail dans le document précité WO-A-2010/105157, un des intérêts de l'élément textile 10 est que la longueur principale de la bande de renfort 1 est facilement ajustable, dans le sens où il suffit pour cela de modifier la distance séparant l'un de l'autre les bords libres 12 et 13, en augmentant ou en diminuant la dimension longitudinale des parties pliées sur elles-mêmes 15 et 16. En effet, par exemple, plus le bord libre 12 est plié en se rapprochant du bord libre 13, plus la dimension principale de la bande 1 décroît. Cet ajustement de taille de la bande 1 est réalisable sur de grandes étendues, sans avoir besoin de la couper.

Comme représenté sur la figure 2, l'élément textile 10 présente une structure spécifique, consistant en un entrelacement de plusieurs monofilaments 17 et de plusieurs fils multifilaments 18. En particulier, comme pour l'exemple représenté à la figure 2, les monofilaments 17 sont entrelacés entre eux, tout en alternant avec les fils multifilaments 18, eux aussi entrelacés entre eux. En pratique, on notera que cette structure textile d'entrelacement des monofilaments 17 et des fils multifilaments 18 se retrouve sur toute la longueur de l'élément textile 10, autrement dit dans sa partie courante 14 et dans ses parties pliées sur elles-mêmes 15 et 16, ainsi qu'au niveau des bords libres 12 et 13, avec la particularité que, au niveau de ces bords libres 12 et 13, les monofilaments 17 et les fils multifilaments 18 sont fusionnés, ce qui présente un intérêt remarquable, explicité plus loin. En pratique, cette fusion au niveau des bords libres 12 et 13 est réalisée par soudage à chaud ou par soudage ultrasonique.

Bien entendu, la fusion des monofilaments 17 et des fils multifilaments 18 au niveau des bords libres 12 et 13 est plus facile à réaliser avant de plier ces bords libres à l'intérieur du volume V, mais on peut envisager d'inverser ces deux étapes de fabrication de l'élément textile 10 ou bien de les réaliser de façon sensiblement concomitante, à l'aide d'une machine ad hoc.

A titre d'exemple de réalisation préférentiel, chacun des monofilaments 17 présente un diamètre compris entre 80 et 500 µm, voire entre 100 et 300 µm, tandis que chacun des fils multifilaments 18 présente un titre compris entre 60 et 1000 deniers, voire entre 100 et 350 deniers.

Selon une forme de réalisation, les monofilaments 17 et les fils multifilaments 18 sont tressés, autrement dit sont entrelacés par tressage, pour fabriquer l'élément textile 10. Dans ce cas, à titre préférentiel, 18 à 128, de préférence 48 à 128, faisceaux de monofilaments 17 sont tressés avec 18 à 128, de préférence 48 à 128, faisceaux de fils multifalements 18. Avantageusement, les spécifications de tressage ci-avant sont combinées aux spécifications dimentionnelles du paragraphe précédent, pour obtenir un élément textile 10 tressé ayant des propriétés remarquables.

Selon une autre forme de réalisation, les monofilaments 17 et les fils multifilaments 18 sont tissés, c'est-à-dire sont entrelacés par tissage, pour fabriquer l'élément textile 10. Ce tissage des monofilaments 17 et des fils multifilaments 18 est notamment réalisé à plat.

Dans tous les cas, la structure textile de l'élément 10, reposant sur l'entrelacement des monofilaments 17 et des fils multifilaments 18, présente, entre autres intérêts, de favoriser la colonisation cellulaire de l'élément textile 10 une fois que la bande de renfort 1 est implantée. En effet, les espaces libres subsistant entre les monofilaments 17 et les fils multifilament 18 constituent, au sein de l'élément textile 10, une porosité de colonisation cellulaire, dans le sens où ces espaces libres sont colonisés par croissance des tissus environnants, notamment par croissance du tissu mou restauré par la bande de renfort 1. L'entrelacement des monofilaments 17 et des fils multifilaments 18 garantit le maintient d'une valeur minimale pour cette porosité, facilitant la colonisation tissulaire de l'élément textile 10, même lorsque la bande 1 est implantée et/ou soumise en service à une tension, en particulier suivant la dimension principale de cette bande.

En pratique, on comprend que les considérations développées juste ci-dessus présentent un intérêt notable pour la partie courante 14 de l'élément textile 10. Ceci étant, comme expliqué ci-après, la structure d'entrelacement des monofilaments 17 et des fils multifilaments 18 présente un intérêt additionnel significatif au niveau des parties pliées sur elles-mêmes 15 et 16 de l'élément textile 10. En effet, comme représenté sur les figures 1 et 3, la bande de renfort 1 est associée à des fils de suture 20 et 30, qui sont rapportés à l'élément textile 10 en étant respectivement attachés aux parties pliées sur elles-mêmes 15 et 16. Ainsi, si on s'intéresse plus spécifiquement au fil de suture 20 visible à la figure 3, étant entendu que le fil de suture 30 présente des aménagements similaires, ce fil de suture 20 inclut une partie 21 agencée au contact de la partie pliée sur elle-même 15 à des fins d'attachement à cette partie pliée sur elle-même 15, le reste du fil 20 formant une partie 22 qui s'étend librement depuis la partie pliée sur elle-même 15. La structure textile d'entrelacement des monofilaments 17 et des fils multifilaments 18 est mise à profit par la partie 21 du fil de suture 20 pour que cette dernière soit attachée à la partie pliée sur elle-même 15 de l'élément textile 10 de manière particulièrement résistante : en effet, la partie 21 du fil de suture 20 coopère alors, aux fins d'attachement, avec quatre épaisseurs superposées d'entrelacement des monofilaments 17 et des fils multifilaments 18, ces quatre épaisseurs superposées présentant une stabilité mécanique, notamment à l'écrasement, suffisante pour que la partie 21 du fil de suture 20 contraigne fortement la partie pliée sur elle-même 15 et ainsi s'y fixe fermement. On comprend au passage l'intérêt que, au niveau des bords libres 12 et 13, les monofilaments 17 et les fils multifilaments 18 soient fusionnés : la jonction amalgamée de ces monofilaments 17 et de ces fils multifilaments 18 évite l'affaiblissement mécanique de la sous-partie intérieure 15.1 de la partie pliée sur elle-même 15, et par là évite l'affaiblissement mécanique de la superposition précitée des quatre épaisseurs de structure textile, sur laquelle agit la partie 21 du fil de suture 20. A titre d'exemple non limitatif, la résistance de l'attachement entre le fil de suture 20 et la partie pliée sur elle-même 15 peut ainsi être supérieure à 300 N.

Par suite, grâce à la haute résistance mécanique de l'attachement entre la partie 21 du fil de suture 20 et l'extrémité de la bande de renfort 1, formée par la partie pliée sur elle-même 15 de l'élément textile 10, la partie 22 du fil de suture 20 est avantageusement utilisée pour la fixation primaire de la bande de renfort vis-à-vis du tissu mou à restaurer : cette partie 22 du fil de suture 20 est par exemple placée autour et/ou passée à travers du tissu mou précité, et/ou est enfilée dans un matériel d'ancrage osseux dont les performances de fixation sont d'ailleurs meilleures lorsque ce matériel coopère avec un tel fil de suture, plutôt qu'avec une portion de bande textile qui, par définition, est plus large que le fil de suture précité. Autrement dit, la partie 22 du fil de suture 20 permet de fixer la bande de renfort 1 à un os par l'intermédiaire d'un matériel d'ancrage qui aurait été difficile, voire impossible à utiliser directement avec l'élément textile 10, eu égard à sa largeur plus importante que celle du fil de suture 20. A titre d'exemple, US-B-7 938 847 divulgue un tel matériel d'ancrage osseux. De surcroît, on conçoit que l'élément textile 10 est plus coûteux à fabriquer que le fil de suture 20, de sorte que la bande de renfort 1 présente l'avantage économique que le chirurgien utilise seulement l'élément textile 10 au contact du tissu mou à restaurer, tandis que le lien entre cet élément textile 10 et un site d'ancrage osseux distant est assuré par la partie 22 du fil de suture 20. De même, en alternative ou en complément aux considérations qui précèdent, la partie 22 du fil de suture 20 est avantageusement utilisée pour manoeuvrer la bande de renfort 1, typiquement en tirant sur ou en tractant cette partie de fil 22, ce qui présente un réel avantage en chirurgie arthroscopique.

En pratique, diverses formes de réalisation sont envisageables en ce qui concerne l'attachement entre les fils de suture 20 et 30 et les parties pliées sur elles-mêmes 15 et 16 de l'élément textile 10. Ainsi, selon une première forme de réalisation, la partie 21 du fil de suture 20 est nouée à la partie pliée sur elle-même 15. Ainsi, dans l'exemple de réalisation considéré à la figure 3, la partie 21 du fil de suture 20 est enroulée à plusieurs reprises autour de la sous-partie extérieure 15.2 de la partie pliée sur elle-même 15, en formant ainsi plusieurs spires adjacentes. On comprend que le nouage obtenu s'apparente, notamment, à un nouage de type « noeud de pendu » ou encore à un nouage de type « Snell ». A titre de variante non représentée, en plus des spires précitées visibles à la figure 3, la partie 21 du fil de suture 20 peut être nouée de manière à former une boucle supplémentaire, à l'intérieur de laquelle sont agencées au moins certaines, voire toutes les spires précitées, à des fins de maintien mécanique relatif de ces dernières.

Une autre forme de réalisation de l'attachement entre les fils de suture et les extrémités correspondantes de la bande de renfort 1 repose sur une couture de ces fils. Ainsi, à titre d'exemple, la figure 4 montre une variante du fil de suture 20, référencée 20', dont la partie 21', fonctionnellement similaire à la partie 21 du fil de suture 20, est cousue directement à la partie pliée sur elle-même 15 de l'élément textile 10. En particulier, la couture de la partie 21' du fil de suture 20' est réalisée en augmentant progressivement le serrage de la partie pliée sur elle-même 15 par la partie de fil 21' cousue, jusqu'à une partie 22' du fil de suture 20', fonctionnellement similaire à la partie 22 du fil de suture 20 : dans ce cas, comme représenté à la figure 4, la partie pliée sur elle-même 15 de l'élément textile 10 présente une forme extérieure étranglée en direction opposée à la partie courante 14 de l'élément textile, par écrasement progressif des quatre épaisseurs de structure textile superposées au niveau de cette partie pliée sur elle-même 15. Le cas échéant, suivant une option de réalisation non représentée, la forme étranglée précitée peut être davantage travaillée pour aboutir à une forme sensiblement tronconique convergente vers la partie de fil 22', notamment par traitement thermique à chaud, éventuellement combiné à une ou des découpes latérales périphériques de la partie pliée sur elle-même 15 à proximité de sa sous-partie coudée 15.3.

Un des autres intérêts de la structure d'entrelacement des monofilaments 17 et des fils multifilaments 18 est de permettre la transmission des contraintes depuis les parties pliées sur elles-mêmes 15 et 16, attachées aux fils de suture 20 et 30, à la partie courante 14 de l'élément textile 10. De la sorte, la résistance à l'arrachement entre les fils de suture 20 et 30 et l'élément textile 10 considéré dans sa globalité est augmentée.

Divers aménagements et variantes à la bande de renfort 1 décrite jusqu'ici sont envisageables. En particulier, le lecteur se reportera au document précité WO-A-2010/105157 pour connaître de tels aménagements. D'ailleurs, dans ce document, des exemples de matériau utilisables pour fabriquer l'élément textile 10 sont fournis, le matériau choisi pouvant d'ailleurs être résorbable ou non. Par ailleurs, à titre optionnel, des agents biologiques et/ou chimiques peuvent être ajoutés à la bande de renfort, notamment par imprégnation de l'élément textile 10 : à titre d'exemple, ces agents sont des facteurs de croissance, des cellules souches, du collagène, ou plus généralement, toutes molécules actives.

## Revendications

1. Bande de renfort (1) pour la restauration d'un tissu mou, tel qu'un tendon ou un ligament,
comprenant un élément textile allongé (10) qui, suivant sa direction longitudinale, présente au moins un bord libre (12, 13) qui est replié dans un volume intérieur (V) de l'élément textile de manière que l'élément textile présente une partie longitudinale pliée sur elle-même (15, 16), laquelle partie pliée sur elle-même forme, en service, une extrémité de la bande de renfort et est attachée à un fil de suture rapporté (20, 30 ; 20') qui est adapté pour être fixé à un os,
**caractérisée en ce que** l'élément textile (10) comprend à la fois des monofilaments (17) et des fils multifilaments (18), qui sont entrelacés les uns avec les autres, en particulier en définissant une porosité de colonisation cellulaire, et qui, au niveau du ou de chaque bord libre (12, 13) de l'élément textile (10), sont fusionnés.

2. Bande de renfort suivant la revendication 1, **caractérisée en ce que** l'élément textile (10) consiste en les monofilaments (17) et les fils multifilaments (18).

3. Bande de renfort suivant l'une des revendications 1 ou 2, **caractérisée en ce que** la ou chaque partie pliée sur elle-même (15, 16) de l'élément textile (10) consiste en l'entrelacement des monofilaments (17) et des fils multifilaments (18).

4. Bande de renfort suivant l'une quelconque des revendications précédentes, **caractérisée en ce que** chaque monofilament (17) présente un diamètre compris entre 80 et 500 µm, de préférence entre 100 et 300 µm, et **en ce que** chaque fil multifilament (18) présente un titre compris entre 60 et 1000 deniers, de préférence entre 100 et 350 deniers.

5. Bande de renfort suivant l'une quelconque des revendications précédentes, **caractérisée en ce que** les monofilaments (17) et les fils multifilaments (18) sont tressés.

6. Bande de renfort suivant la revendication 5, **caractérisée par** 18 à 128, de préférence 48 à 128, faisceaux de monofilaments (17) tressés avec 18 à 128, de préférence 48 à 128, faisceaux de fils multifilaments (18).

7. Bande de renfort suivant l'une quelconque des revendications 1 à 4, **caractérisée en ce que** les monofilaments (17) et les fils multifilaments (18) sont tissés.

8. Bande de renfort suivant l'une quelconque des revendications précédentes, **caractérisée en ce que** le fil de suture (20, 30) est noué à la partie pliée sur elle-même correspondante (15, 16) de l'élément textile (10).

9. Bande de renfort suivant la revendication 8, **caractérisée en ce que** le fil de suture (20, 30) est enroulé autour de la partie pliée sur elle-même correspondante (15, 16), en formant plusieurs spires adjacentes, au moins certaines d'entre elles étant éventuellement maintenues les unes vis-à-vis des autres par une boucle que forme le fil de suture et à l'intérieur de laquelle sont agencées ces spires.

10. Bande de renfort suivant l'une quelconque des revendications 1 à 7, **caractérisée en ce que** le fil de suture (20') est cousu à la partie pliée sur elle-même correspondante (15) de l'élément textile (10).
